# EUROPEAN PATENT SPECIFICATION

(11) **EP 1 648 933 B1**
(45) Date of publication and mention of the grant of the patent: **17.06.2009**
(21) Application number: 04801809.7
(22) Date of filing: 26.07.2004
(51) Int. Cl.: C07K 14/62, C07K 14/765, A61P 3/10, C07K 1/113, A61K 38/28, A61P 5/50

(54) **LONG LASTING INSULIN DERIVATIVES AND METHODS THEREOF**
INSULINDERIVATIVE MIT LANGANHALTENDER WIRKUNG UND VERFAHREN ZU DEREN HERSTELLUNG
DERIVES DE L'INSULINE LONGUE DUREE ET PROCEDES ASSOCIES

(30) Priority: 25.07.2003 US 490110 P; 25.03.2004 US 556585 P
(43) Date of publication of application: 26.04.2006
(60) Divisional application: 09159269.1
(73) Proprietor: ConjuChem Biotechnologies Inc., Montreal, QC H2X 3Y8 (CA)
(72) Inventor: BRIDON, Dominique, P., San Francisco, CA 94114 (US); CASTAIGNE, Jean-Paul, Town of Mount Royal, Québec H3P 1Y6 (CA); HUANG, Xicai, Kirkland, Québec H9J 3X2 (CA); LEGER, Roger, Saint-Lambert, Québec J4R 2V8 (CA); ROBITAILLE, Martin, Granby, Québec J2G 6A2 (CA)
(74) Representative: Williams, Gareth Owen
(86) International application number: PCT/CA2004/001409
(87) International publication number: WO 2005/012346

(56) References cited:
- WO-A-02/04515
- WO-A1-95/05187
- WO-A2-20/05012347
- CA-A1- 2 334 859
- CA-A1- 2 363 712
- US-A- 3 868 356
- US-A- 3 868 357
- US-B1- 6 323 311
- THIBAUDEAU KAREN ET AL: "Synthesis and evaluation of insulin-human serum albumin conjugates" BIOCONJUGATE CHEMISTRY, vol. 16, no. 4, 25 June 2005 (2005-06-25), pages 1000-1008, XP002405343 ISSN: 1043-1802
- JONASSEN ET AL.: 'Fatty acid acylated insulins display protracted action due to binding to serum albumin' PEPTIDE SCIENCE: PRESENT AND FUTURE, PROCEEDINGS OF THE INTERNATIONAL PEPTIDE SYMPOSIUM 1999, KYOTO, pages 674 - 677, XP008062849
- BAUDYS ET AL.: 'Extending insulin action in vivo by conjugation to carboxymethyl dextran' BIOCONJUGATE CHEMISTRY vol. 9, 05 February 1998, pages 176 - 183, XP002363506
- UCHIO ET AL.: 'Site specific insulin conjugates with enhanced stability and extended action profile' ADVANCED DRUG DELIVERY REVIEWS vol. 35, no. 2-3, 1999, pages 289 - 306, XP002363507
- THIBAUDEAU ET AL.: 'Development of novel DAC TM insulin analogues with extended pharmacodynamic profiles' AMERICAN DIABETES ASSOCIATION 64TH SCIENTIFIC SESSIONS 04 June 2004 - 08 June 2004, ORLANDO, FLORIDA, USA, pages A115 - ABSTR.488-P, XP008062845

## Description

### BACKGROUND OF THE INVENTION

### (a) Field of the Invention

This invention relates to a long lasting insulin derivative. More particularly, the insulin derivative comprises an insulin molecule and a reactive group coupled thereto, the reactive group being for covalently bonding a blood component hence generating a long lasting insulin derivative.

### (b) Description of Prior Art

Insulin is a vital endocrine hormone that binds to a cellular surface receptor setting off a cascade of events culminating in glucose absorption from the blood. Impaired levels of insulin lead to severe disorders such as types I and II diabetes. Type I diabetes is a life threatening disease where the patient must daily self-administer multiple doses insulin for survival. Type II diabetes, is also a severe medical disease where the endogenous levels of insulin can no longer maintained correct levels of glycemia because the patient due to a tolerance developed by the patient to endogenous levels of insulin. In order to reduce the onset of long-term consequences, a treatment with insulin becomes necessary after failure in lifestyle changes or when traditional glycemia controlling drugs become ineffective.

Success in the control of glycaemic disorder is highly related with the compliance of patients to the treatment, and reducing the frequency of injection needed is desirable. To do so, it would be highly desirable to be provided with a new long lasting insulin derivative.

Jonassen et al Fatty acid acylated insulins display protracted action due to binding serum albumin' Peptide Science: Present and Future, proceedings of the international peptide symposium (1999) 674-677, discloses an insulin derivative in which a lysine amino acid at position B29 is selectively acylated by fatty acids. As a result, the insulin derivative is found to have an increased binding affinity for human serum albumin.

### SUMMARY OF THE INVENTION

In accordance with the present invention there is provided an insulin derivative comprising an insulin molecule and a reactive group for covalently bonding a blood component, said reactive group being a maleimido-containing group, and wherein the reactive group is coupled to an available amino group of the insulin molecule is selected from the α-amino groups of the N-terminus amino acids of chains A and B, or an ε-amino group of Lys B29.

In a preferred embodiment of the present invention, the insulin molecule is of formula I:

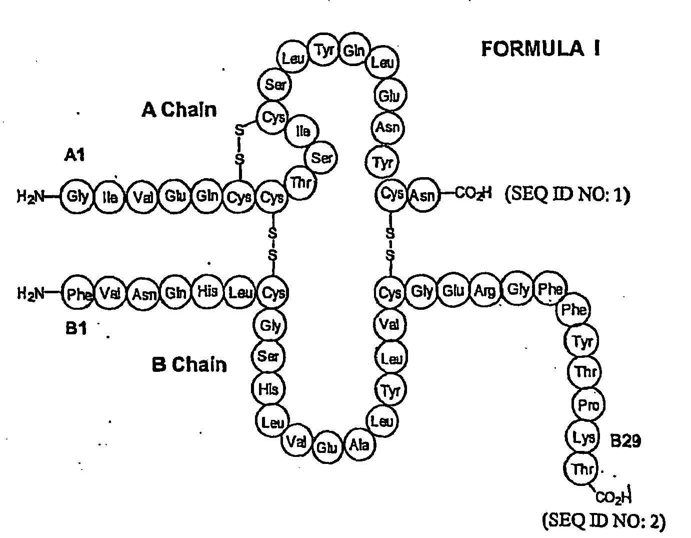

and the reactive group is coupled to an amino acid of the insulin molecule at a position selected from the positions Gly A1, Phe B1 and Lys B29.

In a preferred embodiment of the present invention, the reactive group is MPA (3-MaleimidoPropionic Acid).

In a preferred embodiment of the present invention, the reactive group is coupled to an amino acid of the insulin molecule via a linker, such as, but not limited to ethylenediamine (EDA), 2-[2-(2-amino)ethoxy)] ethoxy acetic acid (AEEA), AEEA-AEEA, -NH₂-(CH₂)ₙ-COOH where n is an integer between 1 and 20 and alkyl chain (C1-C10) motif saturated or unsaturated in which could be incorporated oxygen nitrogen or sulfur atoms, such as, but not limited to glycine, 3-aminopropionic acid (APA), 8-aminooctanoic acid (OA) and 4-aminobenzoic acid (APhA)and combination thereof. ,

In a preferred .embodiment of the present invention, the blood component is a blood protein, more preferably is serum albumin.

In accordance with the present invention, there is provided an insulin conjugate comprising an insulin derivative of the present invention and a blood component, wherein the reactive group and the blood component are conjugated through a covalent bond formed between said reactive group and said blood component. This conjugate is formed *in vivo* or *ex vivo.*

In accordance with the present invention, there is provided a pharmaceutical composition comprising the Insulin derivative of the present invention in association with a pharmaceutically acceptable carrier.

In accordance with the present invention, there is provided a pharmaceutical composition comprising the insulin conjugate of the present invention in association with a pharmaceutically acceptable carrier.

In accordance with the present invention, there is provided a method for treating a glycaemic-related disease or disorder in a subject suffering from said glycaemic-related disease or disorder, comprising administering at least one of the insulin derivatives of the present invention, the conjugate of the present invention and the pharmaceuticals compositions of the present invention to the subject.

### BRIEF DESCRIPTION OF THE DRAWINGS

Fig. 1 illustrates example I to example VIII derived from native human insulin;

Fig. 2 illustrates competitive binding of insulin, insulin derivatives and conjugate of insulin derivatives on liver membranes of wistar rats;

Fig. 3 illustrates competitive binding of insulin, insulin derivatives and conjugate of insulin derivatives on liver membranes of wistar rats;

Fig 4A, 4B, 4C 5A,5B 6A,6B present various phases of 3T3 L1 adipocytes differenciation stages

Fig. 7 illustrates glucose transport in 3T3-L1 adipocytes for insulin, example III and IV and their corresponding conjugate

Fig. 8 illustrates glucose transport in epididymal fat cells from wistar rats adipocytes for insulin, example III and VI and their corresponding conjugate

Fig. 9 illustrates delta glycaemia in function of time in animals treated with insulin at 3.6 mg/kg;

Fig. 10 illustrates delta glycaemia in function of time in animals treated with insulin derivatives of examples I, II and III at 3.6 mg/kg;

Fig 11 illustrates delta glycaemia in function of time in animals treated with insulin derivatives of examples I, II and III at 17.9 mg/kg;

Fig. 12 illustrates delta glycaemia in function of time in animals treated with insulin and insulin derivative of example I at 3.6 mg/kg and insulin derivative of example I at 17.9 mg/kg;

Fig. 13 illustrates delta glycaemia in function of time in animals treated with insulin and insulin derivative of example II at 3.6 mg/kg and insulin derivative of example II at 17.9 mg/kg;

Fig. 14 illustrates delta glycaemia in function of time in animals treated with insulin and insulin derivative of example III at 3.6 mg/kg and insulin derivative of example III at 17.9 mg/kg;

Fig. 15 illustrates the pharmacokinetic profile of native insulin injected subcutaneously and intravenously;

Fig. 16 illustrates the pharmacokinetic profile of conjugate of example III injected subcutaneously and intravenously;

Fig. 17 illustrates the pharmacokinetic profile of example III injected subcutaneously and intravenously;

Fig. 18 illustrates comparative pharmacokinetic profile of insulin, example III and conjugate of example III injected subcutaneously;

Fig. 19 illustrates comparative pharmacokinetic profile of insulin, example III and conjugate of example III injected intravenously;.

Fig. 20 illustrates comparative pharmacodynamic profile post subcutaneous injections of insulin, example I to IV and vehicule in streptozocin induced diabetic rat;

Fig. 21 illustrates comparative pharmacodynamic profile post subcutaneous injections of insulin, the conjugate of example I to IV and vehicule in streptozocin induced diabetic rat;

Fig. 22 illustrates comparative pharmacodynamic profile of repeated subcutaneous injections of example II I (day1 versus day 6 versus day12 versus control); and

Fig. 23 illustrates comparative pharmacodymanic profile of repeated subcutaneous injections of the conjugate of example III (day1 versus day 6 versus day12 versus control).

### DETAILED DESCRIPTION OF THE INVENTION

In accordance with the present invention, there is provided a long lasting insulin derivative. More particularly, the insulin derivative comprises an insulin molecule and a reactive group coupled thereto, the reactive group being for covalently bonding a blood component. In the present application, it is intended that the covalently bonding, resulting in the formation of a conjugate insulin-reactive group -blood component can be formed *in vivo* upon administration of the insulin derivative of the present invention. It is also intended that the covalently bonding can occur *ex vivo* by contacting the insulin derivative of the present invention with a source of albumin that can be recombinant albumin, or extracted from a subject's plasma, or providing from any other suitable source, which source would be known by one skilled in the art.

The insulin molecule may be native human insulin. (see the sequence of native human insulin below in Formula I) or an analogue thereof such as an insulin molecule with amino acid substitution(s), amino acid deletion(s) or amino acid addition(s). The following are listed as examples of insulin analogue that can be used in accordance with the present invention without the intention to limit the present analogue in any way: insulin glargine called Lantus® of Aventis Pharmaceuticals Inc., which has a glycine substituted in position A21 and two residues of arginine added in C-terminus of the chain B; insulin detemir called Levemir® of Novo Nordisk A/S, which is a native human insulin where threonine in position B30 is deleted and tetradecanoyl is added on the lateral chain of lysine B29; insulin lispro called Humalog® of Eli Lilly, which is Lys B28, Pro B29 human insulin; insulin aspart called NovoLog® of Novo Nordisk A/S, which Asp B28 human insulin; and insulin glulisine called Apidra® of Aventis, which is Lys B3, Glu B29 human insulin.

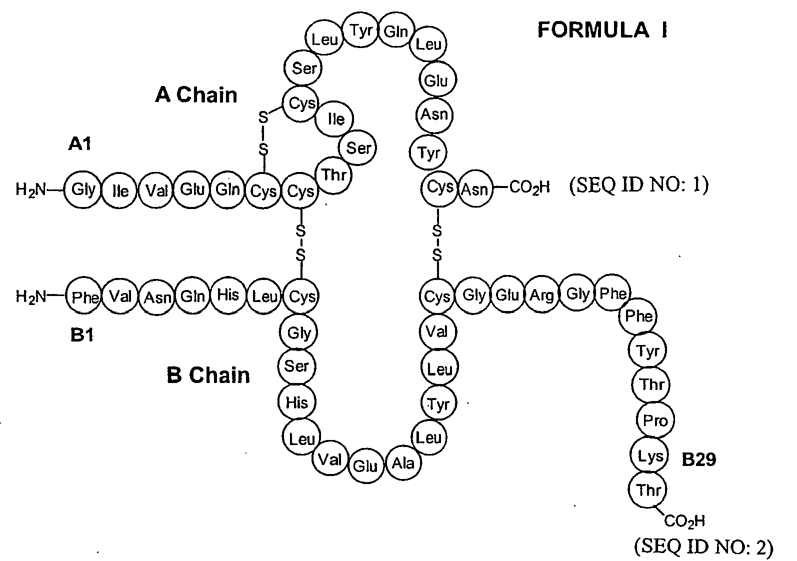

The reactive group may be coupled to different functionalities on the insulin molecule or analogue thereof. Preferably, the reactive group is coupled to an available amino group of the insulin molecule, such as the *a*-amino groups of the N-terminus amino acid of chains A and B, or the ε-amino group of Lys B29. In accordance with the invention, insulin analogue containing substituted and/or added amino acid(s) may contain additional amino group for coupling the reactive group; or other functionalities appropriate for coupling the reactive group thereto. Preferred reactive groups capable to covalently bond a blood component in vivo or ex vivo, are Michael acceptors (α,β, unsaturated carbonyl moiety) succinimidyl-containing groups and maleimido-containing groups. The more preferred reactive group is a maleimido-containing group, and more particularly MPA (3-MaleimidoPropionic Acid).

Optionally, the reactive group is optionally coupled to the insulin molecule via a linker. The linker is preferably selected from the group consisting of hydroxyethyl motifs such as (2-amino) ethoxy acetic acid (AEA), ethylenediamine (EDA), amino ethoxy ethoxy succinimic acid (AEES), 2-[2-(2-amino)ethoxy)] ethoxy acetic acid (AEEA), AEEA-AEEA, - NH2-(CH2)n-COOH where n is an integer from 1 to 20; one or more alkyl chains (C1-C10) saturated or unsaturated in which could be incorporated oxygen nitrogen or sulfur atoms motifs such as glycine, 3-aminopropionic acid (APA), 8-amingoctanoic acid (OA), 4-aminobenzoic acid (APhA). Examples of combinations of linkers include, without limitations, AEEA-EDA, AEEA-AEEA, AEA-AEEA, AEES-AEES, and the like. The preferred linker is 8-aminooctanoic acid (AOA) or the use of no linker with the reactive group MPA. One skilled in the art would readily know what type of linker is suitable for the purpose of the present invention.

The present invention also relates to an insulin conjugate. The conjugate comprises an insulin derivative where its reactive group has reacted with a blood component *in vivo* or *ex vivo* so as to:form a covalent boned. Therefore, the conjugate may be formed *in vivo* by the administration of the Insulin derivative, or *ex vivo* by contacting the insulin derivative to a blood solution or purified blood components *ex vivo* in conditions that allow formation of the covalent bond. Purified blood components can be provided by extraction and purification from blood sample or produced by recombinant techniques. The preferred blood component is a blood protein, and more preferably, serum albumin.

The present invention further relates to method for treating glycaemic-related diseases or disorders, comprising the administration of insulin derivatives or insulin conjugates. Glycaemic-related diseases or disorders include diabetes of Type I and II, and gestational diabetes. Insulin is also known as a growth factor and therefore, the insulin derivatives or insulin conjugates of the present invention can be useful in topical administration for wound healing and other reflated indications.

The following examples are for the purpose of further illustrating the invention as described above rather than for the purpose of limiting the scope of the present invention.

### EXAMPLES

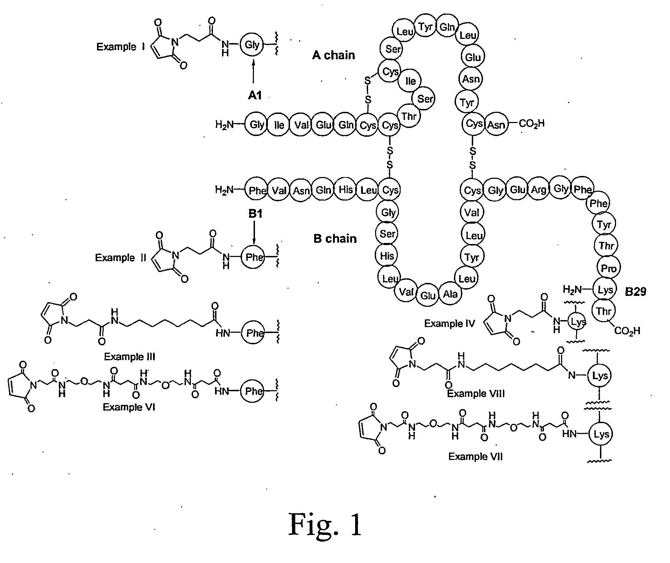

Fig. 1 illustrates the insulin molecule and the Gly A1, Phe B1 and Lys B29 sites referred to along the following examples.

### Example I

### Synthesis of (Gly A1)-MPA-Insulin

Insulin (100 mg) was dissolved in DMF(dimethylformamide) (2 mL) and TFA (100 uL). To the solution, NMM (4-methylmorpholine, 200 uL) and MPA-OSu (N-succinimidyl 3-maleimidopropanoate, 9.2 mg 2.5 equivalents) were added and the reaction was stirred for 2.h. The reaction was quenched by addition of water and adjusted to pH 4 with AcOH(acetic acid). Acetonitrile was added to dissolve the precipitate and the total volume of water/acetonitrile (3:1) was 20 mL. The solution was injected into a semi-preparative HPLC. Phenomenex Luna 10 m phenyl-hexyl 21 mm X 250 mm column equilibrated with an aqueous TFA solution (0.1% TFA in H₂O, solvent A) and an acetonitrile TFA solution (0.1% TFA in acetonitrile, solvent B). Elution was achieved at 9.5 mL/min by running a 27 to 31% B gradient over 120 min. Fractions containing peptides were detected by UV absorbance at 214 and 254 nm. Fractions were collected in 9.5 mL aliquots. Fractions containing the desired product profile were identified by mass detection after direct injection onto LC/MS. The pure fractions at Rt= 36-46 min. were collected, combined and lyophilized to give a white powder (40 mg) along with 23 mg of recovered insulin.

Mass calculated is 5958.5 g/mol, and measured by LC-MS is 5958.0 g/mol.

Table 1 show the amino acid sequence analysis (Edman degradation using phenylisothiocyanate) that was performed to confirm that the A-chain N-terminal was blocked and B-chain N-terminal (phenylalanine) was still free.

**Table 1**

| **Compound** | **Chain** | **Edman Degradation Results (Positions)** | | | |
|---|---|---|---|---|---|
| | | **1** | **2** | **3** | **4** |
| **Human** **Insulin** | **A** | Gly | Ile | Val | Glu |
| | **B** | Phe | Val | Asn | Gln |
| **Example I** | **A** | -- | -- | -- | -- |
| | **B** | Phe | Val | Asn | Gln |
| **Example II** | **A** | Gly | Ile | Val | Glu |
| | **B** | -- | -- | -- | -- |
| **Example III** | **A** | Gly | Ile | Val | Glu |
| | **B** | | -- | -- | |
| **Example IV** | **A** | Gly | Ile | Val | Glu |
| | **B** | Phe | Val | Asn | Gln |

| | | | | | |
|---|---|---|---|---|---|
| Table 1 presents the structural elucidation via Edman degradation of example I to IV | | | | | |

### Example II

### Synthesis of (Phe B1)-MPA-Insulin

Insulin (100 mg) was dissolved in DMSO (dimethylsulphoxide) (4 mL) and Et₃N (triethylamine) (100 uL) with sonication. To the solution, Boc₂O (Di-tert-butyl dicarbonate) (9.3 mg, 2.5 equivalents) was added and the reaction was stirred at ambient temperature for 30 min. The reaction was quenched by addition of water (15 mL) and acetonitrile (5 mL) and the solution was adjusted to pH 4 with AcOH. The solution was injected into a semi-preparative HPLC. Phenomenex Luna 10 m phenyl-hexyl 21 mm X 250 mm column equilibrated with an aqueous TFA solution (0.1% TFA in H2O, solvent A) and an acetonitrile TFA solution (0.1% TFA in acetonitrile, solvent B). Elution was achieved at 9.5 mL/min by running a 27 to 40% B gradient over 120 min. Fractions containing peptides were detected by UV absorbance at 214 and 254 nm. Fractions were collected in 9.5 mL aliquots. Fractions containing the desired product profile were identified by mass detection after direct injection onto LC/MS. Three products (Boc-insulin, Gly A1 Lys B29-BisBoc-insulin and TrisBoc-insulin) were isolated and the fractions of desired (GlyA1 Lys B29)-BisBoc-insulin were combined and lyophilized to give a white powder (72 mg).

(Gly A1 Lys B29)-BisBoc-Insulin (51 mg) in DMF (3 mL) was reacted with MPA-OSu (36 mg) in the presence of Et₃N (30 uL). The reaction was stirred for 2h at ambient temperature. DMF was evaporated by a under vacuum. The residue was treated with TFA (2 mL) for 10 min. and then TFA evaporated. The crude product was dissolved in water/acetonitrile (3:1) and the solution injected into a semi-preparative HPLC. Phenomenex Luna 10 m phenyl-hexyl 21 mm X 250 mm column equilibrated with an aqueous TFA solution (0.1 % TFA in H2O, solvent A) and an acetonitrile TFA solution (0.1% TFA in CH3CN, solvent B). Elution was achieved at 9.5 mL/min by running a 27 to 32% B gradient over 120 min. Fractions containing peptides were detected by UV absorbance at 214 and 254 nm. Fractions were collected in 9.5 mL aliquots. Fractions containing the desired product profile were identified by mass detection after direct injection onto LC/MS. The pure fractions were combined and lyophilized to give a white powder (29 mg).

Mass calculated is 5958.5 g/mol, and measured by LC-MS is 5958.4 g/mol.

The amino acid sequence analysis (Edman degradation using phenylisothiocyanate) was used to confirm that the B-chain N-terminal was blocked and A-chain N-terminal (glycine) was still free(see table 1).

### Example III

### Synthesis of (B1)-MPA-OA-insulin

(Gly A1 Lys B29)-BisBoc-insulin (39 mg) in DMF (3 mL) and Et₃N (30 uL) was reacted with MPA-OA-OSu ([N-succinim idyl 8-N-(3-maleimidopropanylcarbonyl)AminoOctanoate] 25 mg ) for 4 h. DMF was evaporated and the residue treated with TFA for 10 min. After evaporation of TFA, the residue was dissolved in water/acetonitrile (1:3). The solution was injected to a semi-preparative HPLC. Phenomenex Luna 10 m phenyl-hexyl 21 mm X 250 mm column equilibrated with an aqueous TFA solution (0.1% TFA in H2O, solvent A) and an acetonitrile TFA solution (0.1% TFA in acetonitrile, solvent B). Elution was achieved at 9.5 mL/min by running a 27 to 36% B gradient over 120 min. Fractions containing peptides were detected by UV absorbance at 214 and 254 nm. Fractions were collected in 9.5 mL aliquots. Fractions containing the desired product profile were identified by mass detection after direct injection onto LC/MS. The pure fractions were combined and lyophilized to give a white powder (21 mg).

Mass calculated is 6099.5 g/mol, and measured by LC-MS is 6099.6 g/mol.

### Example IV

### Synthesis of (Lys B29)-MPA-insulin

Insulin (74 mg) was dissolved in DMSO (2 mL) and AcOH (46 uL). To the solution Boc₂O (6.9 mg, 2.5 equivalents) was added and the reaction was stirred for 5 h at room temperature. Water (15 mL) and acetonitrile (5 mL) were added and the solution was injected to a semi-preparative HPLC column (C18 phenyl-hexyl) in flow rate of 9.5 mL/min and with gradient from 27-40% over 120 min. The fractions at 43 min. were combined and lyophilized to give (Gly A1 Phe Bl)-Boc2-insulin (30 mg).

(Gly A1 Phe B1)-Boc₂-Insuiin (30 mg) in DMF (2 mL) and NMM (4-methylmorpnoline, 100 uL) was reacted with MPA-OSu (10 mg) for 60 min. DMF was evaporated and the residue treated with TFA for 10 min. The residue was dissolved in water/acetonitrile (3:1) and the solution injected to a semi-preparative HPLC. Phenomenex Luna 10 m phenyl-hexyl 21 mm X 250 mm column equilibrated with an aqueous TFA solution (0.1% TFA in H₂O, solvent A) and an acetonitrile TFA solution (0.1% TFA in acetonitrile, solvent B). Elution was achieved at 9.5 mL/min by running a 27 to 32% B gradient over 120 min. Fractions containing peptides were detected by UV absorbance at 214 and 254 nm. Fractions were collected in 9.5 mL aliquots. Fractions containing the desired product profile were identified by mass detection after direct injection onto LC/MS. The pure fractions were combined and lyophilized to give a white powder (22.2 mg).

Mass calculated is 5958.5 g/mol, and measured by LC-MS is 5958.0 g/mol.

The amino acid sequence analysis (Edman degradation using phenylisothiocyanate) was used to confirm that both B-chain (phenylalanine) and A-chain (glycine) N-termini were free (see table 1).

### Example V

### Synthesis of MPA-(AEES)₂-COOH linker

Flash column chromatography was carried out using a Biotage® "40i flash chromatography" modular system. Semi preparative HPLC purifications were done on a Waters "Breeze" system 1500 series using a Phenomex luna (RP-18, 10 u phenyl-hexyl 250 X 21.2 mm) column with a 9.5 mL/min mobile phase flow rate. A Gilson 690 system was used for preparative scale purification using a Phenomex luna (RP-18, 10 u phenyl-hexyl 250 X 50.0 mm) column with a 50 mL/min mobile phase flow rate. A Gradient of acetonitrile (CH₃CN) (0.1%TFA) in water (0.1%TFA) was used with further details indicated in each compound's synthetic procedure. LC-MS was performed using an Agilent 1100 series LC-MSD single quadrupole mass spectrometer with an ES1 electrospray source.

### MPA-(AEES)₂-COOH linker synthesis

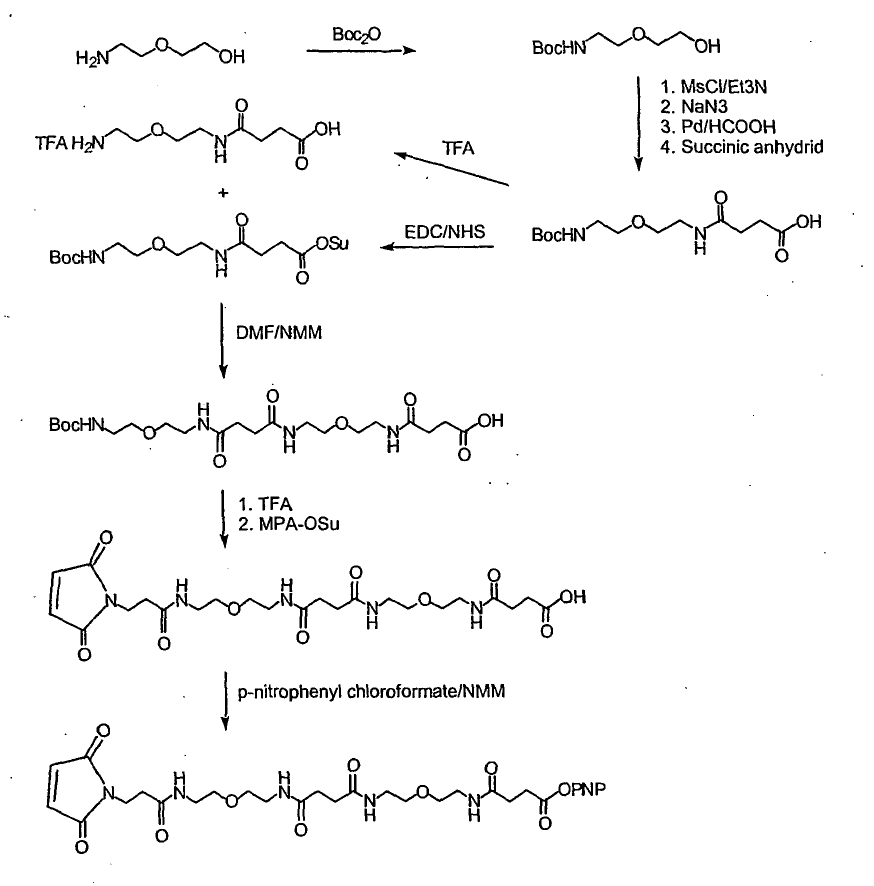

### Example VI

### Synthesis of (Phe B1)-MPA-(AEES)₂-Insulin

2-(2-Aminoethoxy)ethanol (50.0 g) in methanol (150 mL) was reacted with Boc₂O (93.4 g) for 30 min. The methanol was evaporated in vacuo and the residue was taken up in ethyl acetate, washed with water, brine and dried with sodium sulfate. After evaporation of the solvent, the crude product was used for the next step. MS m/z 205.

The protected alcohol was dissolved in N,N-dimethyl formamide (500 mL) in the presence of Et₃N (66 mL). MsCl (Mesylate chloride) (33.4 mL) was added dropwise at 0°C over 30 min and then stirred at ambient temperature for 1 h. NaN₃ (127.6 g) was then added to the reaction mixture followed by NMM (N-methylmorpholine, 215 mL) and the reaction was stirred at 40-50°C for 16 h. The reaction mixture was poured into ethyl acetate (2L) and washed with water. The water layer was back extracted with ethyl acetate (2L) and the combined ethyl acetate layers were washed with water, brine and dried. After evaporation of the solvent, the crude product was used for the next step (101.4 g contains some DMF). MS m/z 231.

The crude product (62.4 g) was dissolved in methanol (300 mL) followed by the addition of Pd(OAc)₂ (3.0g) and formic acid (96% 62 g). After completion of the reaction, Pd species were removed by filtration through celite. The methanol was removed in vacuo and dried further under vacuum. The crude product was used in the next step.

The crude product was dissolved in dichloromethane and neutralized by Et₃N until it is basic. Succinic anhydride (32.3 g) was added in one portion. The reaction was stirred at ambient temperature for 1 h. The solvent was removed in vacuo and the residue acidified by HCl 1 N to pH 3. The product was extracted with ethyl acetate. The ethyl acetate layer was passed through a silica gel plug (1 kg). Then the silica gel was washed with 2-4% methanol in ethyl acetate. The pure fractions (as judged by thin layer chromatography) were combined and the solvent removed in vacuo to give Boc-AEES as an oily residue (36 g, 44%). MS m/z 305

Boc-AEES (5.5 g) was treated with N-hydroxysuccinimide (NHS, 4.57 g) and ethyl-(dimethylaminopropyl)carbodiimide hydrochloride (EDC, 7.62 g) in dichloromethane (30 mL) for 2 h. The NHS ester was poured into ethyl acetate (500 mL), washed with 0.1 N HCl and dried with sodium sulfate. The solvent was removed in vacuo and the residue used as such in the next step.

Boc-AEES (6.05 g) was treated with trifluoroacetic acid (TFA, 10 mL) for 10 min. TFA was removed in vacuo and dried further under vacuum. The (AEES) amino acid in was dissolved in N,N-dimethylformamide (20 mL) and basified with excess NMM. The crude product from example V was then added and the reaction was stirred at ambient temperature for 1 h. The solvent was evaporated under reduced pressure and the residue was injected into preparative HPLC using a 5-40% gradient over 60 min. The solvent was removed and the residue dried under vacuum to give Boc-(AEES)₂-COOH as an oil (5,64 g, 84%). MS m/z 478.

Boc-(AEES)₂-COOH (3.60 g) was treated with trifluoroacetic acid (TFA, 10 mL) for 10 min. TFA was removed in vacuo and dried further under vacuum. The (AEES)₂ amino acid in was dissolved in N,N-dimethylformamide and basified with NMM. MPA-OSu (2.94 g) was added and the mixture was stirred for 30 min. N,N-dimethylformamide was removed under vacuum. The residue was dissolved in water and injected into preparative HPLC using a 5-40% gradient over 60 min. The pure fraction were combined and the solvent was removed to give MPA-(AEES)₂-COOH as an off-white solid (3.8 g, 95%). MS m/z 542.2.

MPA-(AEES)₂-COOH (3.04 g) was dissolved in N,N-dimethylformamide (20 mL) and the presence of NMM (1.13 mL) and treated with p-nitrophenyl chloroformate (1.13 g). The reaction mixture was stirred at ambient temperature for 2 h. N,N-dimethylformamide was removed under vacuum. The residue was purified by flash column chromatography using Biotage® system. The column was rinsed with ethyl acetate (500 mL) followed by 10% methanol in ethyl acetate (1 L). The pure fractions were combined and the solvent removed to give MPA-(AEES)₂-CO₂PNP as a solid (1.39 g, 37%). MS m/z 663.

Gly A1, Lys B29-BisBoc-insulin (200 mg) in N,N-dimethylformamide (10 mL) was coupled with MPA-(AEES)₂-CO₂PNP from example VIII (200 mg) in the presence of NMM (200 uL) at ambient temperature for 16 h. N,N-dimethylformamide was removed under vacuum and the residue treated with TFA for 10 min. TFA was removed in vacuo, the residue was dissolved in water and injected into HPLC using a 27-32% gradient over 120 min. The pure fractions were combined and the solvent lyophilized to give ( Phe B1)-MPA(AEES)₂-Insulin as a white powder (95.0 mg, 43.7%). MS m/z 6330.4.

### Example VII

### Synthesis of (B29)-MPA(AEES)2-Insulin

Gly A1 Phe B1-BisBoc-Insulin (200 mg) in N,N-dimethylformamide (10 mL) was coupled with MPA-(AEES)₂-CO2PNP as from Example VI (200 mg) in the presence of NMM (200 uL) at ambient temperature for 16 h. N,N-dimethylformamide was removed under vacuum and the residue treated with TFA for 10 min. After TFA was removed in vacuo, the residue was dissolved in in water and injected into HPLC using a 27-32% gradient over 120 min. The pure fractions were combined and the solvent lyophilized to give ( Lys B29)-MPA(AEES)₂-Insulin as a white powder (56.3 mg, 25.9%). MS m/z 6328.8.

### Example VIII

### Synthesis of (B29)-MPA(OA)-Insulin

Gly A1 Phe B1-BisBoc-Insulin (205 mg) in N,N-dimethylformamide (10 mL) was coupled with MPA-OA-CO₂Su (139 mg) in the presence of NMM (20 uL) at ambient temperature for 16 h. The solvent was removed under vacuum and the residue treated with TFA for 10 min. After TFA was removed in vacuo, the residue was dissolved in in water and injected into HPLC using a 27-36% gradient over 120 min. The pure fractions were combined and the solvent lyophilized to give ( Lys B29)-MPA(OA)-Insulin as a white powder (64.2 mg, 31%). MS m/z 6098.8.

### Example IX

### In vitro binding assays

Liver membranes of Wistar rats were incubated with [1251] insulin and increasing concentrations of insulin, DAC:insulin as described in Fig. 1 and their corresponding conjugate for 16 hours at 4°C. The membranes were filtered and washed 3 times and the filters were counted to determine [1251] insulin specifically bound. IC50 were calculated using GraphPad Prism software.

The results of IC50 are illustrated in Table 2.

**Table 2**

| | IC50 (nM) |
|---|---|
| Insulin | 12.0 |
| Example I | 57.1 |
| Example II | 18.8 |
| Example III | 17.2 |
| Example IV | 46.0 |
| Example VI | 24.9 |
| Example VII | 58.1 |
| Example VIII | 64.1 |
| Conjugate of Example I | 2059.0 |
| Conjugate of Example II | 100.5 |
| Conjugate of Example III | 87.7 |
| Conjugate of Example IV | 1190.0 |
| Conjugate of Example VI | 38.5 |
| Conjugate of Example VII | 508.2 |
| Conjugate of Example VIII | 1013.4 |

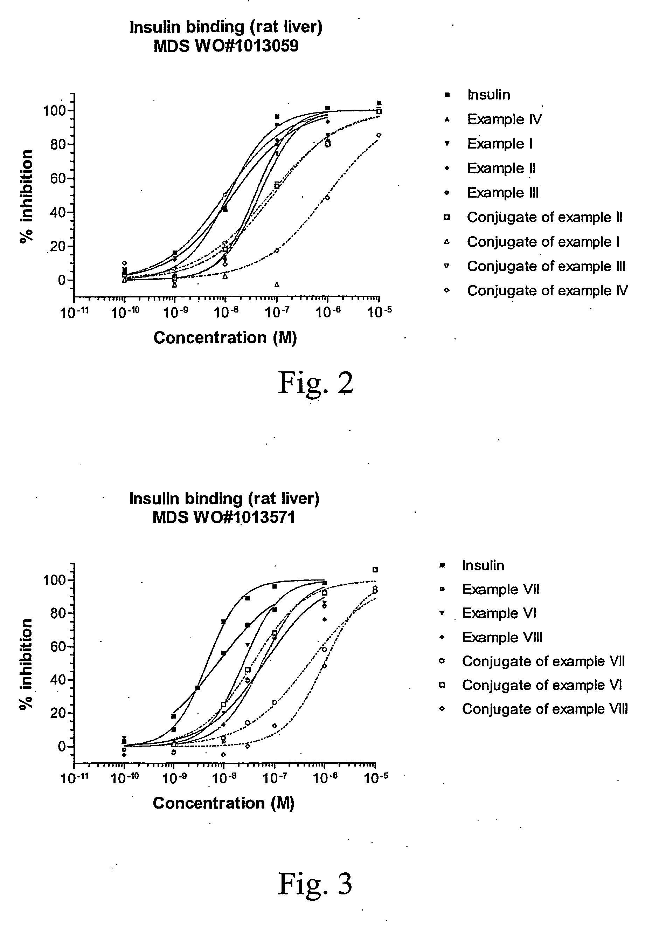

Fig. 2 and Fig. 3 illustrates the insulin binding in rat liver membrane in terms of the % of inhibition in function of the concentration of the insulin derivative of the present invention.

### Example X

### In vitro bioactivity

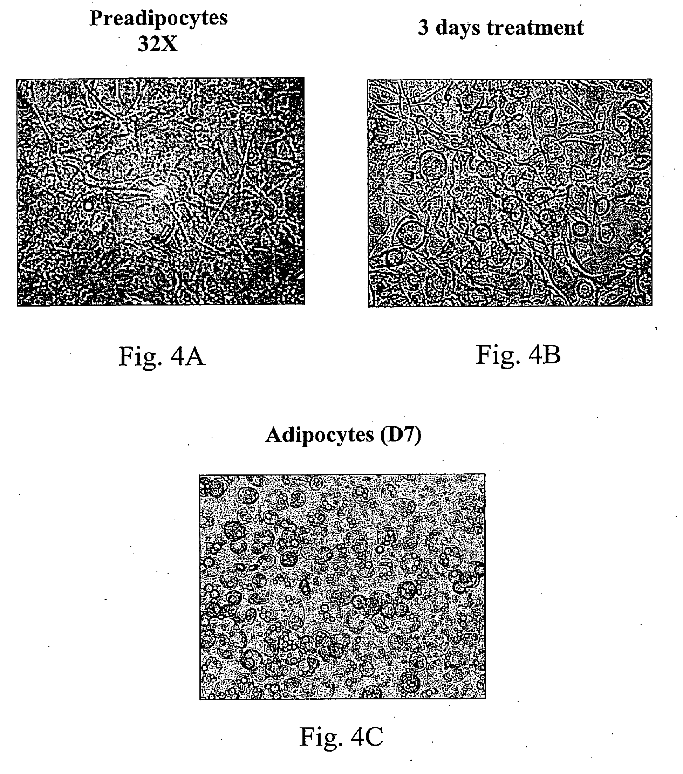

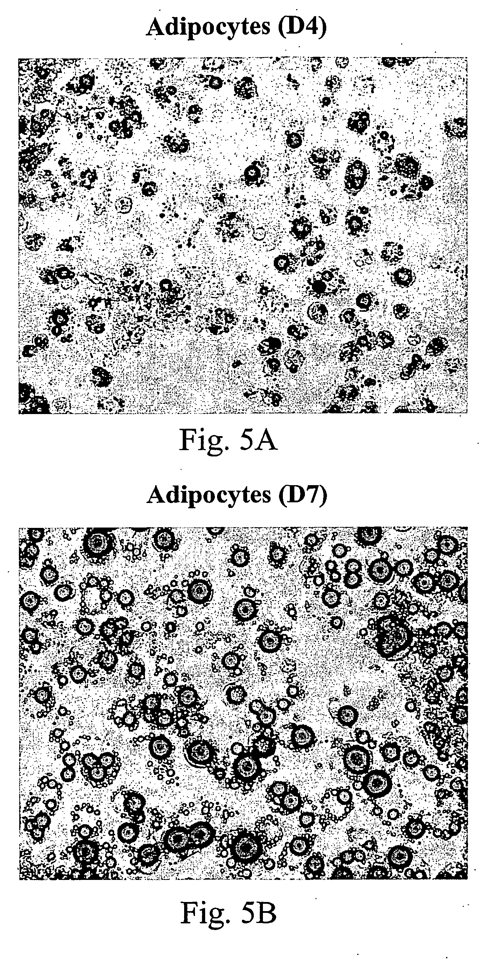

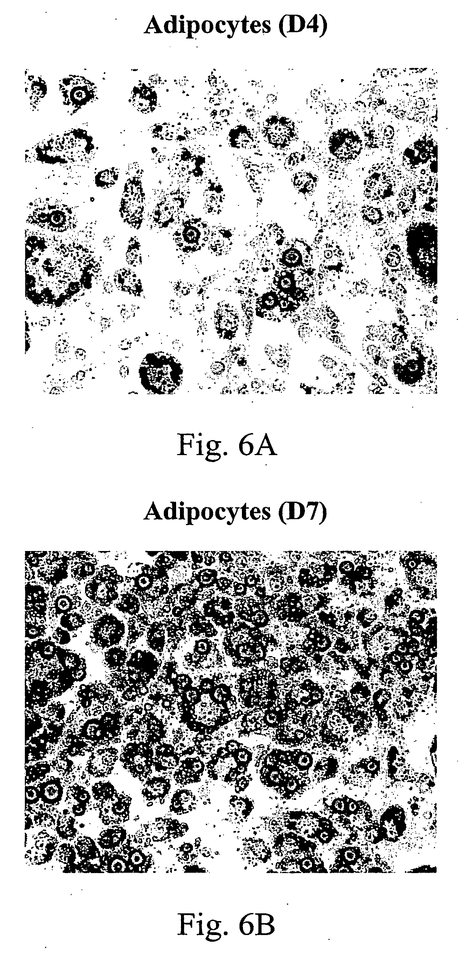

Glucose uptake in adipocytes was used to evaluate the in vitro activity. 3T3-L1 cells, a murine fibroblast cell line was differenciated in adipocytes for used in the bioassay. 3T3-L1 cells were plated and grown to confluency in DMEM and 10% FBS, followed by an incubation for two days. Differentiation was induced by adding dexamethasone and insulin (D0). By day 7, more than 90% of the cells displayed an adipocyte phenotype, i.e. accumulation of lipid droplets. Figs. 4A-4C show preadipocytes, cells after 3 days and adipocytes at 7 days. Figs. 5A and 5B show oil red o staining of adipocytes at 4 days and adipocytes at 7 days. Figs. 6A and 6B show oil red o and methylene blue staining at 4 days and at 7 days.

3T3-L1 adipocytes were starved overnight in DMEM containing 5mM glucose and 0.5% FBS. Cells were rinsed in Kreb's-Ringer-Hepes buffer containing 1 % BSA and incubated with increasing concentrations of insulin, DAC:insulin derivatives and their corresponding conjugate for 20 minutes at 37°C and with [¹⁴C]-2-deoxy-D-glucose (1µCi/well) for an additional 20 minutes. Cells were solubilized and radioactivity was measured. Glucose uptake (%) was calculated versus insulin control and EC50 were calculated using GraphPad Prism^{™} software.

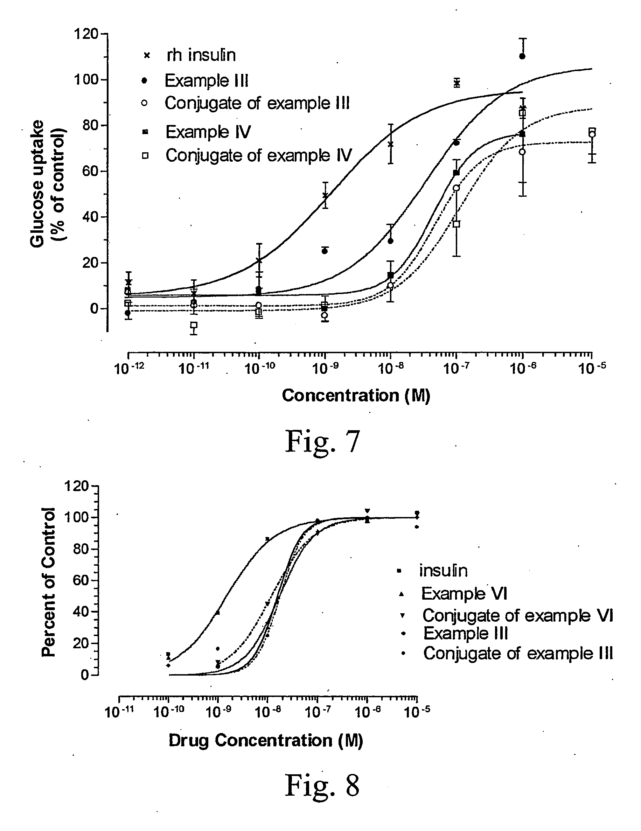

Table 3 show the EC50 results for the compounds tested and Fig. 7 illustrates the glucose uptake in % of control in function of the concentration (M) of the compounds.

**Table 3**

| **Compound** | **EC50 (nM)** |
|---|---|
| Human insulin | 1.2 |
| Example III Conjugate of Example III | 34 48 |
| Example IV Conjugate of Example IV | 45 110 |

### Example XI

In addition, glucose uptake in an other source of adipocytes was used to evaluate the in vitro activity. Epididymal fat obtained from Wistar derived male rats weighing 175 ± 25 g is used. The tissue (0.03 g/ml) is degraded by collagenase in modified HEPES solution pH 7.4 at 37°C. Test compound and/or vehicle is incubated with 500 µl aliquots in modified HEPES buffer pH 7.4 and D-[3-3H]Glucose (2.5 µCi/ml) is then added for 2 hour incubation. Test compound-induced the increase of glucose incorporation by more than 50 percent or more (≥50%) relative to the control 2 nM insulin response, indicates possible insulin receptor agonist activity. Test compound inhibition of the insulin-induced glucose incorporation response by more than 50% indicates insulin receptor antagonist activity. Compounds are screened at 10,1, 0.1, 0.01 and 0.001 µM.

Table 4 show the EC50 results for the compounds tested and Fig. 8 illustrates the glucose uptake in % of control in function of the concentration (M) of the compounds.

**Table 4**

| **Compound** | **EC50 (nM)** |
|---|---|
| Human insulin | 1.4 |
| Example III Conjugate of Example III | 17.5 17.8 |
| Example VI Conjugate of Example VI | 15.4 13.3 |

### Example XII

### In vivo experiments

Evaluation the blood glucose lowering efficacy of recombinant human insulin versus and insulin derivatives of the present invention when administered subcutaneously to diabetic female db/db mice, are compared.

Tested compounds were administered by a single subcutaneous bolus injection in 5-6 week-old female db/db mice weighing 24.3 to 33.3 g. The average volume of dosing solution injected was 0.35 mL/mouse (12.5 mL/kg).

Recombinant (E. coli) human insulin (called herein below "rH insulin") is provided by ICNTM at a concentration of 28 IU/mg.

Stock solutions of insulin derivatives were prepared at 14.29 mg/mL (~ 400 IU/mL) by reconstituting the synthesized insulin derivatives with acidified water (- pH 2). Stock solutions were subsequently diluted with 0.9% NaCl and 0.22 µm filtered (Millex GV) to obtain the dosing solutions shown in Table 5. Group 1. received 0.9% NaCl USP as control solution.

**Table 5**

| **Groups** | **Tested Compounds** | **Actual Solution Concentration** **(µg/mL)** |
|---|---|---|
| 2 | rH Insulin | 0.29 |
| 3 | Example I | 0.29 |
| 4 | Example I | 1.43 |
| 5 | Example II | 0.29 |
| 6 | Example II | 1.43 |
| 7 | Example III | 0.29 |
| 8 | Example III | 1.43 |

Groups and treatments are summarized in the Table 6.

**Table 6**

| **Groups** | **Test/Control Articles** | **Dose Level** **(µg/kg)** | **Dose Equivalence** ***(~IU/kg)** | **Number of Animals** **(females)** |
|---|---|---|---|---|
| 1 | 0.9% NaCi | 0 | 0 | 5 |
| 2 | rH insulin | 3.6 | 100 | 5 |
| 3 | Example I | 3.6 | 100 | 5 |
| 4 | Example I | 17.9 | 500 | 5 |
| 5 | Example II | 3.6 | 100 | 5 |
| 6 | Example II | 17.9 | 500 | 5 |
| 7 | Example III | 3.6 | 100 | 5 |
| 8 | Example III | 17.9 | 500 | 5 |

| | | | | |
|---|---|---|---|---|
| * Based on the potency of the rH insulin estimated at 28 IU/mg by the vendor. | | | | |

Blood sampling (one drop) was performed via the tail tip and glucose levels were determined using a hand-held glucometer (Model: One Touch UltraTM, Lifescan Canada). Blood glucose levels were determined from all animals once prior to administration (pre-dose), and at 1, 2, 3, 4, 6, 24, 30, 48 and 72 hours post-dose.

### In vivo results:

All animals appeared normal prior to administration of the tested compounds. Approximately one hour post-dose, Group 2 of animals treated subcutaneously with 100 IU/kg of recombinant human insulin (rH insulin), exhibited slight decrease in activity and uncoordinated gait. Other treated animals appeared normal throughout the experiment. A slight decrease of food consumption was observed in animals treated with 17.9 mg /kg of compound of Example III.

Table 7 shows food consumption (total weight/cage (g)) following a single administration of rH insulin and insulin derivatives.

**Table 7**

| **Food consumption: total weight/cage (g)** | | | | | | |
|---|---|---|---|---|---|---|
| **Groups** | **Treatment** | **24h pre-dose** | **0-6h** | **0-24h** | **24-48h** | **48-72h** |
| 1 | 0.9% NaCl | 30.4 | 6.2 | 30.7 | 28.9 | 31.6 |
| 2 | rH insulin 3.6 mg/kg | 30.2 | 6.6 | 29.2 | 30.7 | 31.7 |
| 3 | Example I 3.6 mg/kg | 28.7 | 5.8 | 30.5 | 28.8 | 28.7 |
| 4 | Example I 17.9 mg/kg | 29.8 | 5.1 | 28.3 | 31.3 | 31.5 |
| 5 | Example II 3.6 mg/kg | 31.8 | 6.4 | 30.3 | 29.8 | 31.0 |
| 6 | Example II 17.9 mg/kg | 30.5 | 6.4 | 31.2 | 26.5 | 29.7 |
| 7 | Example III 3.6 mg/kg | 29.9 | 6.0 | 31.4 | 31.9 | 31.8 |
| 8 | Example III 17.9 mg/kg | 28.7 | 4.5 | 27.0 | 22.8 | 24.5 |

Table 8 shows food consumption versus control (total weight/cage (g)) following a single administration of rH insulin and insulin derivatives.

**Table 8**

| **% Food consumption versus control group** | | | | | | |
|---|---|---|---|---|---|---|
| **Groups** | **Treatment** | **24h pre-dose** | **0-6h** | **0-24h** | **24-48h** | **48-72h** |
| 1 | 0.9% NaCl | 100.0 | 100.0 | 100.0 | 100.0 | 100.0 |
| 2 | rH insulin 3.6 mg/kg | 99.3 | 106.5 | 95.1 | 106.2 | 100.3 |
| 3 | Example I 3.6 mg/kg | 94.4 | 93.5 | 99.3 | 99.7 | 90.8 |
| 4 | Example I 17.9 mg/kg | 98.0 | 82.3 | 92.2 | 108.3, | 99.7 |
| 5 | Example II 3.6 mg/kg | 104.6 | 103.2 | 98.7 | 103.1 | 98.1 |
| 6 | Example II 17.9 mg/kg | 100.3 | 103.2 | 101.6 | 91.7 | 94.0 |
| 7 | Example III 3.6 mg/kg | 98.4 | 96.8 | 102.3 | 110.4 | 100.6 |
| 8 | Example III 17.9 mg/kg | 94.4 | 72.6 | 87.9 | 78.9 | 77.5 |

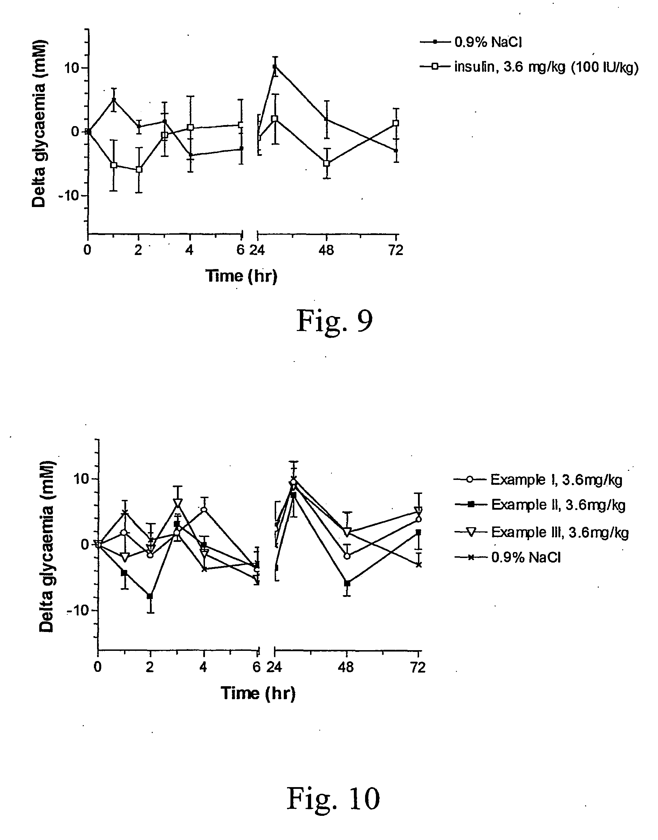

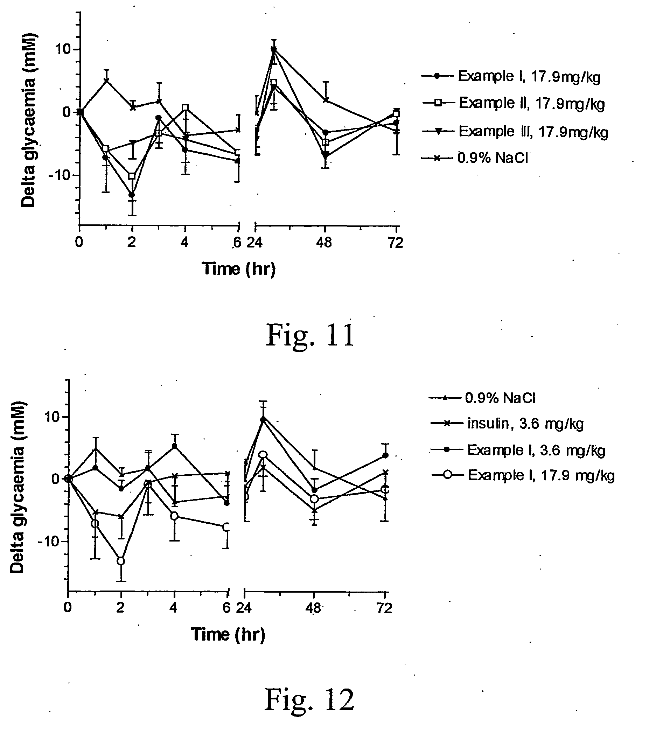

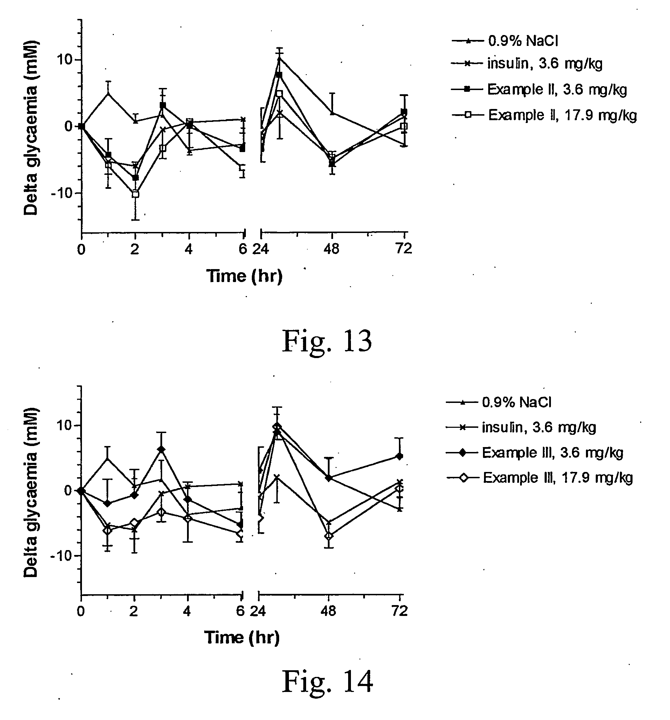

Delta glycaemia is calculated from blood glucose levels of post-dose glucose level versus the pre-dose glucose level for each individual mouse are reported in Figs 9, 10, 11, 12, 13, and 14. In general, insulin derivatives (Example I, Example II and Example III) were able to lower blood glucose concentrations in a dose-dependent manner and recombinant insulin, tested at one dose level only (100 IU/kg), was active for 2 hours. At 3.6 mg/kg, Example 2 was as active as insulin during the first 2 hours while only a marginal effect was observed with Example I and Example III. The lowering effect of rH insulin was more pronounced at 17.9 mg/kg since it was observed for up to 24 hours. Although the overall picture tends to demonstrate that insulin derivatives were active for up to 24 hours (as compared to the control group), it is important to note that glucose levels decreased at 1-2 hours post-dose then increased at 3-4 hours and decreased again at 6 hours. This 'up and down' response might indicate that the feeding habits and the metabolism of the mice are important parameters that can affect the efficacy of the drug. It is important to mention that db/db mice develop insulin resistance with age, which could explain the very high dose of insulin that had to be injected to observe glucose lowering effect.

### Example XIII

### Pharmacokinetic profile in normal rats

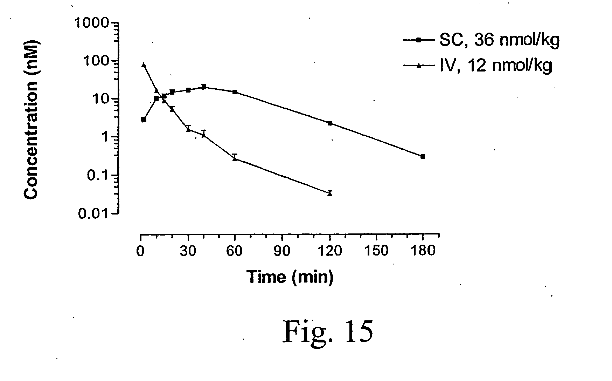

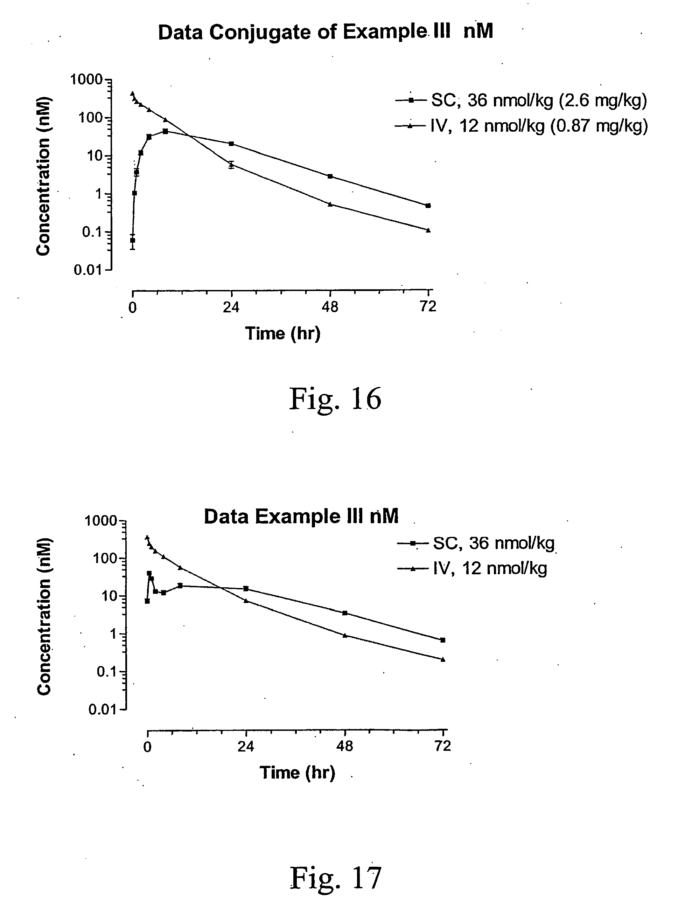

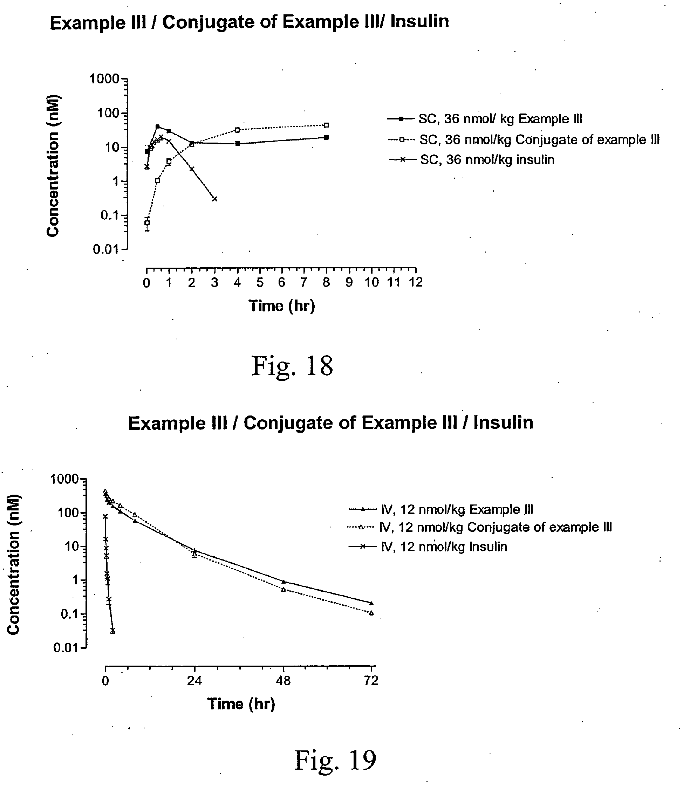

Rh insulin, the insulin derivative of example III and the conjugate of the insulin derivative of Example III were administered to 7-8 week-old male CD rats either at 36 hmol/kg sc or 12 nmol/kg intravenously (iv). Blood samples were collected up to 72 hours (only up to 3 hours for rh insulin-treated animals). The drug levels were determined using a human insulin ELISA kit (Linco). The pharmacokinetic parameters were calculated by non-compartmental analysis using the WinNonlin software, N=4 rats per compound/route. Fig. 15 is the pharmacokinetic profile of insulin in normal SD rats where insulin was administered sc at 36 nmol/kg and iv at 12 nmol/kg. Fig. 16 is the pharmacokinetic profile of the conjugate of example III in normal SD rats where the conjugate was administered sc at 36 nmol/kg and iv at 12 nmol/kg. Fig. 17 is the pharmacokinetic profile of the insulin derivative of example III in normal SD rats where the insulin derivative was administered sc at 36 nmol/kg and iv at 12 nmol/kg. Fig. 18 is the subcutaneous PK profile of the administration of insulin, insulin derivative of example III and the conjugate of the insulin derivative of example III. Fig. 19 is the intravenous PK profile of the administration of insulin, insulin derivative of example III and the conjugate of the insulin derivative of example III.

### Example XIV

### Single dose pharmacodynamic in diabetic rats

Diabetes was induced in male CD rats with a single i.v. injection of streptozotocin (60 mg/kg). Two days later, rats received a single sc injection of DAC™ :Insulin derivatives at 120 nmol/kg, preformed conjugates at 300 nmol/kg, rh insulin at 20U/kg (120 nmol/kg) or vehicle. Blood glucose level were measured with a hand-held glucometer just prior injection and a 1, 2, 3, 4, 6, 8, 10, 11, 24, 30 and 48 hours postdose with 5 rats/groups except for vehicle were 3 rats/ group were tested. Glycemia of normal rats ranged from 5.2 to 7.6 mmol/L.

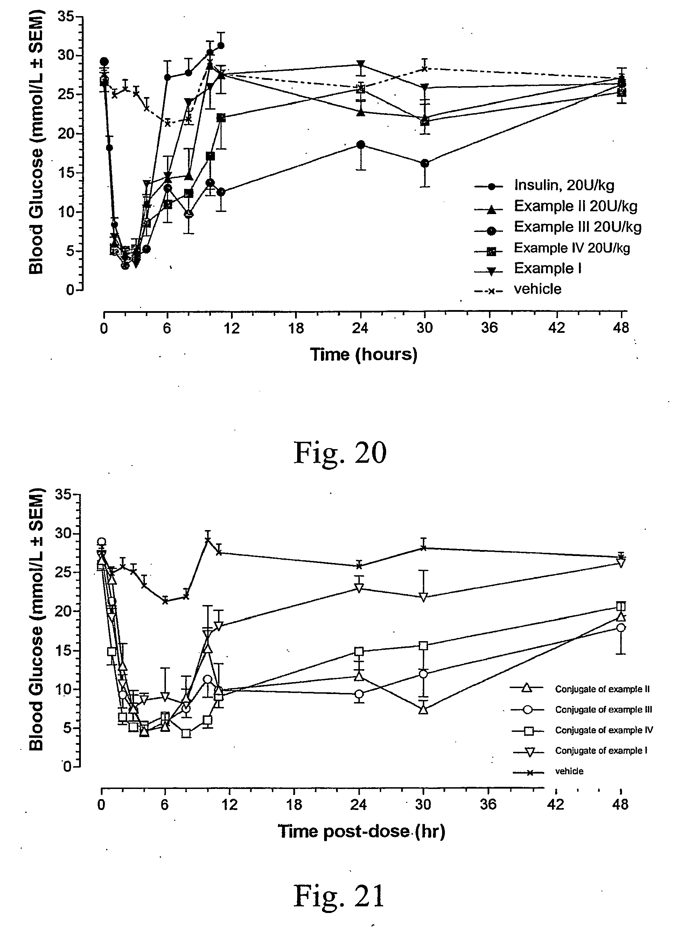

Figs. 20 shows the blood glucose level in rats after administration of 120nmol/kg of example I to IV. Fig 21 shows the blood glucose level in rats after administration of the corresponding conjugate of example I to IV of the present invention at 300nmol/kg.

### Example XV

### Evaluation of the potency of a DAC:Insulin derivative and its corresponding preformed conjugate following repeated subcutaneous administration

This assay was conducted to evaluate the potency of the insulin derivative of the example III and its conjugate versus free human recombinant insulin following repeated subcutaneous injections in adult male CD® rats.

Type 1 diabetes was induced in male CD® rats on study day 1 by a single intravenous (i.v.) injection of streptozotocin (60 mg/kg, pH 4.5). Hyperglycemia was confirmed using a blood glucose monitor to test blood. Test compound was administered once daily on study days 3 through 14 at 1 mL/kg body weight by subcutaneous (s.c.) injection. Blood glucose levels were tested just prior to dose administration each day and at 2,8 and 18 hours following administration. Feed and water consumption were monitored daily. Body weights were collected on study days 1, 3, 6, 9, 12, 15 and 17.

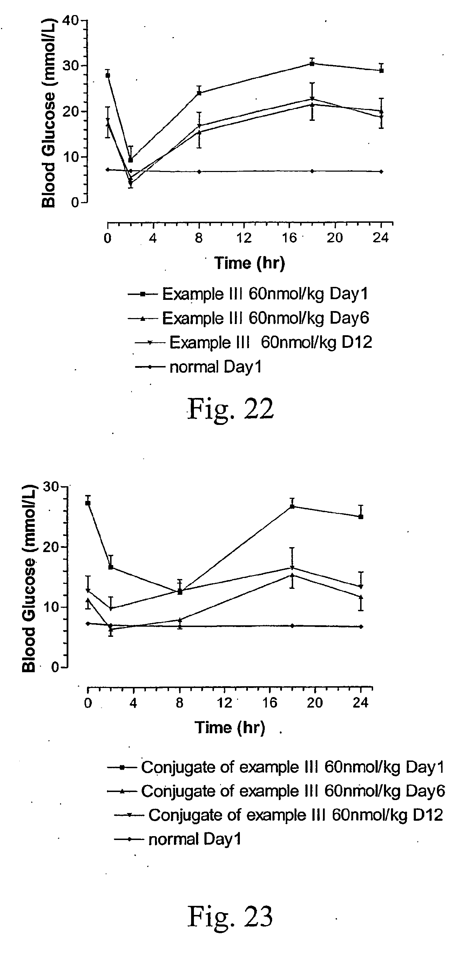

Fig. 22 illustrates the blood glucose daily profile at day 1, 6 and 12 for the insulin derivative of example III and Fig. 23 illustrates the blood glucose daily profile at day 1, 6 and 12 for the conjugate of the insulin derivative of example III.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

## Claims

1. An insulin derivative comprising an insulin molecule and a reactive group for covalently bonding a blood component, said reactive group being a maleimido-containing group, and wherein the reactive group is coupled to an available amino group of the insulin molecule selected from the α-amino groups of the N-terminus amino acids of chains A and B and the ε-amino group of Lys B29.

2. The insulin derivative of claim 1, wherein the available amino group is the ε-amino group of Lys B29.

3. The insulin derivative of claim 1, wherein the available amino group is the α-amino group of Gly A1 or Phe B1.

4. The insulin derivative of any preceding claim, wherein the insulin molecule is selected from native human insulin or an insulin molecule with amino acid substitution(s), amino acid deletion(s) or amino acid addition(s).

5. The insulin derivative of any preceding claim, wherein the insulin molecule is of formula I: 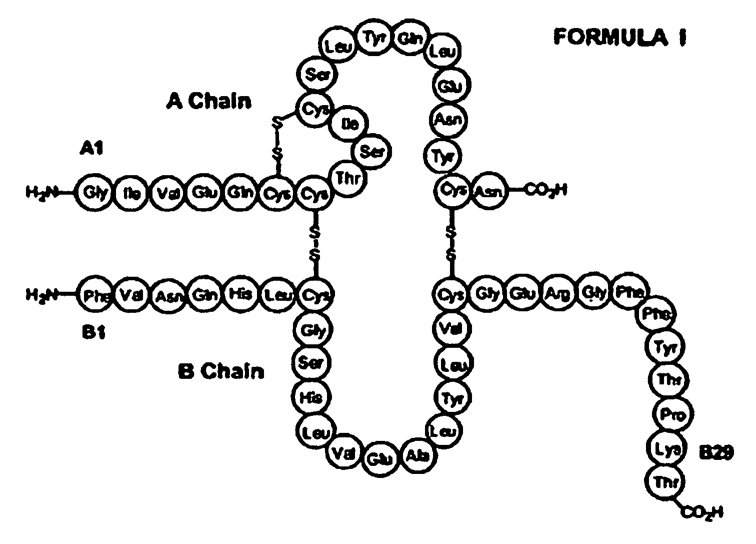 and the reactive group is coupled to an amino acid of the insulin molecule at a position selected from the positions Gly A1, Phe B1 and Lys B29.

6. The insulin derivative of any of claims 1 to 5, wherein the reactive group coupled to the available amino group of the insulin molecule is:

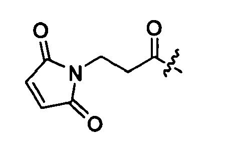

7. The insulin derivative of any preceding claim, wherein the reactive group is coupled to the available amino group of the insulin molecule via a linker.

8. The insulin derivative of claim 7, wherein the reactive group is coupled to the available amino group of the insulin molecule by reacting a linker with the reactive group and the available amino group of the insulin molecule, and wherein the linker is selected from the group consisting of ethylenediamine (EDA), 2-[2-(2-amino) ethoxy)] ethoxy acetic acid (AEEA), AEEA-AEEA and NH₂-(CH₂)ₙ-COOH where n is an integer between 1 and 20.

9. The insulin derivative of claim 7, wherein said linker is:

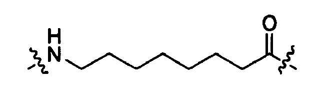

10. The insulin derivative of claim 5, wherein the insulin molecule is coupled at the terminal Gly of A1 with 3-maleimidopropanamide, and wherein the α-amino group of Gly is the amide nitrogen of the 3-maleimidopropanamide.

11. The insulin derivative of claim 5, wherein the insulin molecule is coupled at the terminal Phe of B1 with 3-maleimidopropanamide, and wherein the α-amino group of Phe is the amide nitrogen of the 3-maleimidopropanamide.

12. The insulin derivative of claim 5, wherein the insulin molecule is coupled at the terminal Phe of B1 with 8-N-(3-maleimidopropanylcarbonyl)aminooctanamide, and wherein the α-amino group of Phe is the amide nitrogen of the octanamide of 8-N-(3-maleimidopropanylcarbonyl)aminooctanamide.

13. The insulin derivative of claim 5,wherein the insulin molecule is coupled at the B29 Lys with 3-maleimidopropanamide and wherein the ε-amino group of Lys is the amide nitrogen of the 3-maleimidopropanamide.

14. The insulin derivative of any preceding claim, wherein said blood component is a blood protein.

15. The insulin derivative of claim 14, wherein said blood protein is serum albumin.

16. An insulin conjugate comprising an insulin derivative according to any one of claims 1 to 15 and a blood component, wherein the reactive group is covalently bonded to the blood component.

17. The insulin conjugate of claim 16, wherein the blood component is a blood protein.

18. The insulin conjugate of claim 17, wherein the blood protein is serum albumin.

19. The insulin conjugate of claim 16, wherein said conjugate is formed *ex vivo.*

20. The insulin conjugate of claim 17, wherein said blood protein is recombinant albumin.

21. The insulin conjugate of claim 16, wherein said blood component is produced by recombinant techniques.

22. Use of the derivative of any one of claims 1 to 15, or the conjugate of any one of claims 16 to 21, for the preparation of a medicament for the treatment of a glycaemic-related disease or disorder.

23. The use as claimed in claim 22, wherein said glycaemic-related disease or disorder is selected from the group consisting of diabetes, diabetes of type I, diabetes of type II and gestational diabetes.

24. The use as claimed in claim 22, wherein the glycaemic-related disease or disorder is selected from the group consisting of diabetes of type I and diabetes of type II.

25. The derivative of any of claims 1 to 15, or the conjugate of any one of claims 16 to 21, for use in the treatment of glycaemic-related diseases or disorders.

26. The derivative or conjugate as claimed in claim 25, wherein said glycaemic-related disease or disorder is selected from the group consisting of diabetes, diabetes of type I, diabetes of type II and gestational diabetes.

27. The derivative or conjugate as claimed in claim 25, wherein the glycaemic-related disease or disorder is selected from the group consisting of diabetes of type I and diabetes of type II.

## Patentansprüche

1. Insulinderivat, welches ein Insulinmolekül umfasst und eine reaktive Gruppe zur kovalenten Bindung einer Blutkomponente, wobei die reaktive Gruppe eine Maleimido-enthaltende Gruppe ist, und worin die reaktive Gruppe an eine verfügbare Aminogruppe des Insulinmoleküls gekoppelt ist, ausgewählt aus den α-Aminogruppen der N-terminalen Aminosäuren der Ketten A und B und der ε-Aminogruppe von Lys B29.

2. Das Insulinderivat nach Anspruch 1, wobei die verfügbare Aminogruppe die ε-Aminogruppe von Lys B29 ist.

3. Das Insulinderivat nach Anspruch 1, wobei die verfügbare Aminogruppe die α-Aminogruppe von Gly A1 oder Phe B1 ist.

4. Das Insulinderivat nach einem der vorangehenden Ansprüche, wobei das Insulinmolekül ausgewählt wird aus nativem Humaninsulin oder einem Insulimmolekül mit Aminosäure-Subsitution(en), Aminosäure-Deletion(en) oder Aminosäure-Addition(en).

5. Das Insulin-Derivat nach einem der vorangehenden Ansprüche, wobei das Insulinmolekül die folgende Formel I aufweist: 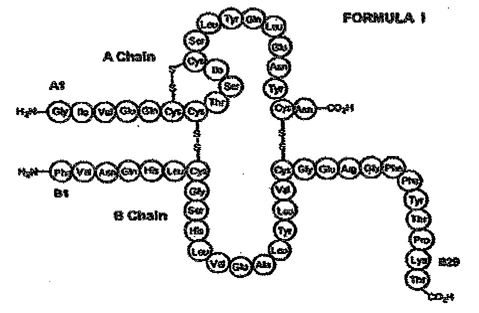 und die reaktive Gruppe an eine Aminosäure des Insulinmoleküls gekoppelt ist an eine Position, ausgewählt aus den Positionen Gly A1, Phe B1 und Lys B29.

6. Das Insulin-Derivat nach einem der Ansprüche 1 bis 5, wobei die reaktive Gruppe, welche an die verfügbare Aminogruppe des Insulinmoleküls gekoppelt ist, besteht aus:

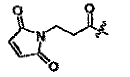

7. Das Insulin-Derivat nach einem der vorangehenden Ansprüche, wobei die reaktive Gruppe an die verfügbare Aminogruppe des Insulinmoleküls über einen Linker gekoppelt ist.

8. Das Insulin-Derivat nach Anspruch 7, wobei die reaktive Gruppe an die verfügbare Aminogruppe des Insulinmoleküls gekoppelt ist durch Umsetzung eines Linkers mit der reaktiven Gruppe und der verfügbaren Aminogruppe des insulinmoleküls, und wobei der Linker ausgewählt ist aus der Gruppe bestehend aus Ethylendiamin (EDA), 2-[2-(2-Amino)ethoxy)]ethoxyessigsäure (AEEA), AEEA-AEEA und NH₂-(CH₂)ₙ-COOH, wobei n eine ganze Zahl zwischen 1 und 20 ist.

9. Das Insulin-Derivat nach Anspruch 7, wobei der Linker besteht aus:

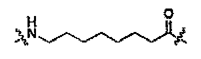

10. Das Insulin-Derivat nach Anspruch 5, wobei das Insulinmolekül an dem terminalen Gly von A1 gekoppelt ist mit 3-Maleimidopropanamid, und worin die α-Aminogruppe von Gly der Amidstickstoff von 3-Maleimidopropanamid ist.

11. Das Insulin-Derivat nach Anspruch 5, wobei das Insulinmolekül an dem terminalen Phe von B1 gekoppelt ist mit 3-Maleimidopropanamid, und worin die α-Aminogruppe von Phe der Amidstickstoff von 3-Maleimidopropanamid ist.

12. Das Insulinderivat nach Anspruch 5, wobei das Insulinmolekül an dem terminalen Phe von B1 gekoppelt ist mit 8-N-(3-Maleimidopropanylcarbonyl)aminooctanamid und wobei die α-Aminogruppe von Phe der Amidstickstoff des Octanamids von 8-N-(3-Maleimidopropanylcarbonyl)aminooctanamid ist.

13. Das Insulinderivat nach Anspruch 5, wobei das Insulinmolekül an dem B29 Lys gekoppelt ist mit 3-Maleimidopropanamid, und wobei die ε-Aminogruppe von Lys der Amidstickstoff des 3-Maleimidopropanamid ist.

14. Das Insulinderivat nach einem der vorangehenden Ansprüche, wobei die Blutkomponente ein Blutprotein ist.

15. Das Insulinderivat nach Anspruch 14, wobei das Blutprotein Serumalbumin ist.

16. Insulin-Konjugat, welches ein Insulinderivat nach einem der Ansprüche 1 bis 15 und eine Blutkomponente umfasst, wobei die reaktive Gruppe kovalent an die Blutkomponente gebunden ist.

17. Das Insulin-Konjugat nach Anspruch 16, wobei die Blutkomponente ein Blutprotein ist.

18. Das Insulin-Konjugat nach Anspruch 17, wobei das Blutprotein Serumalbumin ist.

19. Das Insulin-Konjugat nach Anspruch 16, wobei das Konjugat ex vivo gebildet wird.

20. Das Insulin-Konjugat nach Anspruch 17, wobei das Blutprotein rekombinantes Albumin ist.

21. Das Insulin-Konjugat nach Anspruch 16, wobei die Blutkomponente durch rekombinante Verfahren hergestellt wurde.

22. Verwendung des Derivates nach einem der Ansprüche 1 bis 15, oder des Konjugates nach einem der Ansprüche 16 bis 21, zur Bereitung eines Medikaments zur Behandlung einer glykämisch-bedingten Erkrankung oder Störung.

23. Die Verwendung, wie in Patentanspruch 22 beansprucht, wobei die glykämisch-bedingte Erkrankung oder Störung ausgewählt wird aus der Gruppe bestehend aus Diabetes, Diabetes vom Typ I, Diabetes vom Typ II und Schwangerschaftsdiabetes.

24. Die Verwendung, wie in Patentanspruch 22 beansprucht, wobei die glykämisch-bedingte Erkrankung oder Störung ausgewählt wird aus der Gruppe bestehend aus Diabetes vom Typ I und Diabetes vom Typ II.

25. Das Derivat nach einem der Ansprüche 1 bis 15, oder das Konjugat nach einem der Ansprüche 16 bis 21, zur Verwendung bei der Behandlung von glykämisch-bedingten Erkrankungen oder Störungen.

26. Das Derivat oder das Konjugat, wie in Patentanspruch 25 beansprucht, wobei die glykämisch-bedingte Erkrankung oder Störung ausgewählt wird aus der Gruppe bestehend aus Diabetes, Diabetes vom Typ I, Diabetes vom Typ II und Schwangerschaftsdiabetes.

27. Das Derivat oder Konjugat, wie in Patentanspruch 25 beansprucht, wobei die glykämisch-bedingte Erkrankung oder Störung ausgewählt wird aus der Gruppe bestehend aus Diabetes vom Typ I und Diabetes vom Typ II.

## Revendications

1. Dérivé de l'insuline comprenant une molécule d'insuline et un groupe réactif en vue de former une liaison covalente avec un composant sanguin, ledit groupe réactif étant un groupe contenant du maléimide et dans lequel le groupe réactif est couplé à un groupe aminé disponible sur la molécule d'insuline, choisi parmi les groupes α-aminés des acides aminés de l'extrémité amino-terminale des chaînes A et B, et le groupe ε-aminé de la Lys B29.

2. Dérivé de l'insuline selon la revendication 1, dans lequel le groupe aminé disponible est le groupe ε-aminé de la Lys B29.

3. Dérivé de l'insuline selon la revendication 1, dans lequel le groupe aminé disponible est le groupe α-aminé de la Gly A1 ou de 1a Phe B1.

4. Dérivé de l'insuline selon l'une quelconque des revendications précédentes, dans lequel la molécule d'insuline est choisie à partir de l'insuline humaine naturelle ou d'une molécule d'insuline portant une ou plusieurs substitutions d'acide aminé, une ou plusieurs délétions d'acide aminé, ou bien une ou plusieurs additions d'acide aminé.

5. Dérivé de l'insuline selon l'une quelconque des revendications précédentes, dans lequel la molécule d'insuline est de formule 1: 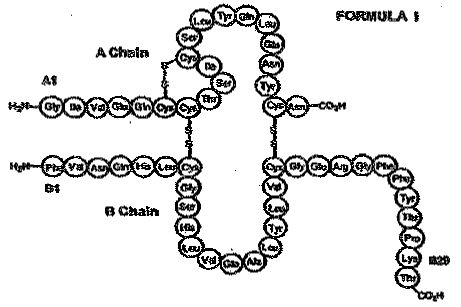 et le groupe réactif est couplé à un acide aminé de à molécule d'insuline en une position choisie parmi les positions de la Gly A1 de la Phe B1 et de la Lys B29.

6. Dérivé de l'insuline selon l'une quelconque des revendications 1 à 5, dans lequel le groupe réactif couplé au groupe aminé disponible sur la molécule d'insuline est:

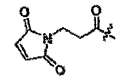

7. Dérivé de l'insuline selon l'une quelconque des revendications précédentes, dans lequel le groupe réactif est couplé au groupe aminé disponible sur la molécule d'insuline par l'intermédiaire d'un adaptateur.

8. Dérivé de l'insuline selon la revendication 7, dans lequel le groupe réactif est couplé au groupe aminé disponible sur la molécule d'insuline par réaction d'un adaptateur avec le groupe réactif et le groupe aminé disponible sur la molécule d'insuline, et dans lequel l'adaptateur est choisi dans le groupe comprenant l'éthylène-diamine (EDA), l'acide 2-[2-(2-amino)éthoxy)]éthoxyacétique (AEEA), l'AEEA-AEEA et le NH₂-(CH₂)ₙ-COOH, n étant un nombre entier compris entre 1 et 20.

9. Dérivé de l'insuline selon la revendication 7, dans lequel ledit adaptateur est:

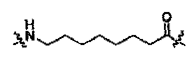

10. Dérivé de l'insuline selon la revendication 5, dans lequel la molécule d'insuline est couplée sur la Gly terminale de A1 avec du 3-maléimidopropanamide, et dans lequel le groupe α-aminé de la Gly est l'azote amidé du 3-maléimidopropanamide.

11. Dérivé de l'insuline selon la revendication 5, dans lequel la molécule d'insuline est couplée sur la Phe terminale de B1 avec du 3-maléimidopropanamide, et dans lequel le groupe α-aminé de la Phe est l'azote amidé du 3-maléimidopropanamide.

12. Dérivé de l'insuline selon la revendication 5, dans lequel la molécule d'insuline est couplée sur la Phe terminale de B1 avec du 8-N-(3-maléimidopropanylcarbonyl)aminooctanamide, et dans lequel le groupe α-aminé de la Phe est l'azote amidé de l'octanamide de 8-N-(3-maléimidopropanylcarbonyl)aminooctanamide.

13. Dérivé de l'insuline selon la revendication 5, dans lequel la molécule d'insuline est couplée sur la Lys de B29 avec du 3-maléimidopropanamide, et dans lequel le groupe ε-aminé de la Lys est l'azote amidé du 3-maléimidopropanamide.

14. Dérivé de l'insuline selon l'une quelconque des revendications précédentes, dans lequel ledit composant sanguin est une protéine sanguine.

15. Dérivé de l'insuline selon la revendication 14, dans lequel ladite protéine sanguine est la sérum-albumine.

16. Conjugué de l'insuline comprenant un dérivé de l'insuline selon l'une quelconque des revendications 1 à 15 et un composant sanguin, dans lequel le groupe réactif est lié de manière covalente au composant sanguin.

17. Conjugué de l'insuline selon la revendication 16, dans lequel le composant sanguin est une protéine sanguine.

18. Conjugué de l'insuline selon la revendication 17, dans lequel la protéine sanguine est la sérum-albumine.

19. Conjugué de l'insuline selon la revendication 16, dans lequel ledit conjugué est formé *ex vivo.*

20. Conjugué de l'insuline selon la revendication 17, dans lequel ladite protéine sanguine est une albumine recombinée.

21. Conjugué de l'insuline selon la revendication 16, dans lequel le composant sanguin est produit par des techniques de recombinaison.

22. Emploi du dérivé selon l'une quelconque des revendications 1 à 15, ou du conjugué selon l'une quelconque des revendications 16 à 21, en vue de la préparation d'un médicament pour le traitement d'une maladie ou d'un trouble lié la glycémie.

23. Emploi tel que revendiqué dans la revendication 22, dans lequel ladite maladie ou ledit trouble lié à la glycémie est choisi dans le groupe comprenant les diabètes, les diabètes de type I, les diabètes de type II et les diabètes gestationnels.

24. Emploi tel que revendiqué dans la revendication 22, dans lequel la maladie ou le trouble lié à la glycémie est choisi dans le groupe comprenant les diabètes de type I et les diabètes de type II.

25. Dérivé selon l'une quelconque des revendications 1 à 15, ou conjugué selon l'une quelconque des revendications 16 à 21, en vue de l'emploi pour le traitement de maladies ou de troubles liés la glycémie.

26. Dérivé ou conjugué tel que revendiqué dans la revendication 25, dans lequel ladite maladie ou ledit trouble lié à la glycémie est choisi dans le groupe comprenant les diabètes, les diabètes de type I, les diabètes de type II et les diabètes gestationnels.

27. Dérivé ou conjugué tel que revendiqué dans la revendication 25, dans lequel la maladie ou le trouble lié à la glycémie est choisi dans le groupe comprenant les diabètes de type I et les diabètes de type II.
